# EUROPEAN PATENT APPLICATION

(11) **EP 4 465 038 A1**
(43) Date of publication of application: **20.11.2024**
(21) Application number: 24176066.9
(22) Date of filing: 15.05.2024
(51) Int. Cl.: G01N 30/88

(54) **ANALYSIS SYSTEM**

(30) Priority: 18.05.2023 JP 2023082578
(71) Applicant: ARKRAY, Inc., Kyoto-shi, Kyoto 601-8045 (JP)
(72) Inventor: DEMURA, Tomohiro, Kyoto, 602-0008 (JP); SUZUKI, Shunichi, Kyoto, 602-0008 (JP)
(74) Representative: Dehns

(57) **Abstract**

An analysis system includes: an analyzer (30) that analyzes a specimen, the analyzer being switchable between a first measurement mode and a second measurement mode that includes a measurement item different from a measurement item of the first measurement mode; and a management unit (80) that selects a measurement mode from the first measurement mode and the second measurement mode of the analyzer based on information on a clinical department that has requested a test to be set for the specimen.

## Description

### BACKGROUND

### Technical Field

The present invention relates to an analysis system.

### Related Art

As apparatuses that analyze hemoglobin in blood, analysis apparatuses using liquid chromatography are known (see Japanese Patent Application Laid-Open (JP-A) No. 2017-198666 and JP-A No. 2019-60655).

A test content of a specimen containing blood often varies depending on a clinical department that requests a test. Further, specimens having different test contents are delivered in a mixed manner to a laboratory where the specimens are tested. A worker in the laboratory sets a measurement mode of an analysis apparatus according to a test content of a specimen. In a case in which the measurement mode is set according to the test content of the specimen as described above, it is necessary to pay attention to prevent occurrence of a setting error, and a burden on the worker tends to increase.

### SUMMARY

At least preferred embodiments of the invention provide an analysis system capable of suppressing an increase in burden on a test worker even when specimens having different test contents exist in a mixed manner in a configuration of analyzing the specimens using an analyzer having a plurality of measurement modes.

An analysis system according to an aspect of the invention includes:
an analyzer that analyzes a specimen, the analyzer being switchable between a first measurement mode and a second measurement mode that includes a measurement item different from a measurement item of the first measurement mode; and
a management unit that selects a measurement mode from the first measurement mode and the second measurement mode of the analyzer based on information on a clinical department that has requested a test to be set for the specimen.

According to the analysis system of the embodiment of the invention, it is possible to suppress the increase in burden on the test worker even when the specimens having different test contents exist in a mixed manner in the configuration of analyzing the specimens using the analyzer having the plurality of measurement modes.

### BRIEF DESCRIPTION OF THE DRAWINGS

Exemplary embodiments will be described in detail based on the following figures, wherein:
Fig. 1 is a schematic configuration diagram of an analysis apparatus of an embodiment;
Fig. 2 is a configuration diagram of a control system of an analysis system of the embodiment;
Fig. 3 is a flowchart of a liquid chromatography measurement program;
Fig. 4 is a flowchart of a specimen measurement mode selection program; and
Fig. 5 is a configuration diagram of a control system of an analysis system of a modified example.

### DETAILED DESCRIPTION

Hereinafter, an analysis system of an embodiment of the invention will be described. Note that dimensions, materials, shapes, relative arrangements, and the like of constituent parts described in the embodiment are not particularly limited unless otherwise specified. It is not intended to limit the technical scope only thereto.

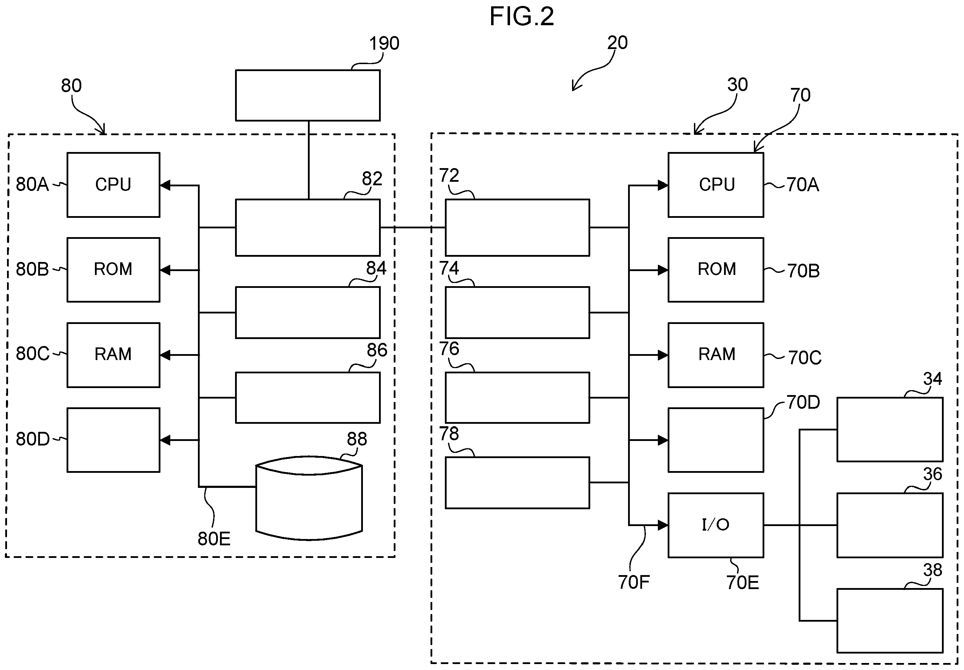

As illustrated in Fig. 2, an analysis system 20 of the present exemplary embodiment includes an analysis apparatus 30 and a management apparatus 80.

### (Analysis Apparatus 30)

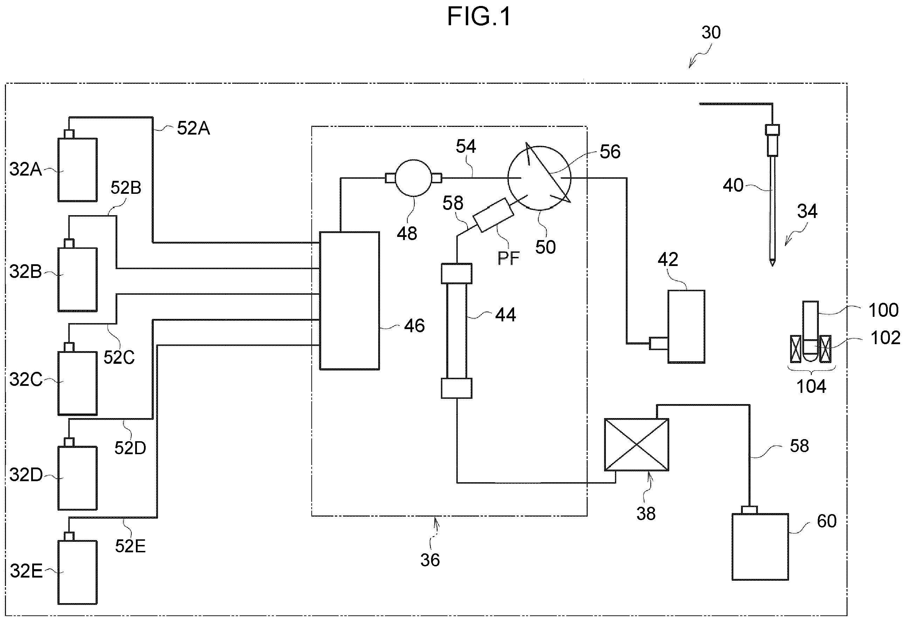

Fig. 1 illustrates a schematic configuration of the analysis apparatus 30.

The analysis apparatus 30 is an apparatus that analyzes a component of a specimen. Specifically, the analysis apparatus 30 is an apparatus that measures the concentration of HbA1c in blood using high performance liquid chromatography (HPLC). Note that the analysis apparatus 30 is an example of an analyzer of the invention. In addition or alternatively, the analyzer of the invention may be an analysis apparatus that analyzes another component of the specimen (biological sample).

As illustrated in Fig. 1, the analysis apparatus 30 includes, as an example, a plurality of (five in Fig. 1) eluent bottles 32A, 32B, 32C, 32D, and 32E.

The eluent bottles 32A, 32B, 32C, 32D, and 32E are bottles respectively holding eluents A, B, C, D, and E that need to be supplied to an analytical column 44 which will be described later. For example, each of the eluents has different composition, component ratio, pH, osmotic pressure, or the like depending on the use.

In addition, the analysis apparatus 30 includes, as an example, a sample preparation unit 34, an analysis unit 36, and a photometric unit 38.

The sample preparation unit 34 is a unit having a function of preparing a sample to be introduced into the analytical column 44 from a blood sample 102 collected from a blood collection tube 100. The sample preparation unit 34 includes, for example, a nozzle 40 and a dilution tank 42. The nozzle 40 is configured to collect various liquids including the blood sample 102 from the blood collection tube 100. The dilution tank 42 is a tank configured to dilute the blood sample 102, and is supplied with the blood sample 102 collected by the nozzle 40.

The analysis unit 36 is a unit having a function of controlling adsorption and desorption of biological components to and from a packing material of the analytical column 44 and providing various biological components to the photometric unit 38. The analysis unit 36 includes the analytical column 44, a manifold 46, a liquid feeding pump 48, and an injection valve 50.

The analytical column 44 is an instrument holding the packing material configured to selectively adsorb hemoglobin in the blood sample 102.

The manifold 46 is a part that selectively supplies an eluent from a specific eluent bottle among the plurality of eluent bottles 32A, 32B, 32C, 32D, and 32E to the analytical column 44. The manifold 46 is connected to the eluent bottles 32A, 32B, 32C, 32D, and 32E via pipes 52A, 52B, 52C, and 52E, respectively, and is connected to the injection valve 50 via a pipe 54.

The liquid feeding pump 48 is a pump configured to apply power for moving an eluent to the injection valve 50. The liquid feeding pump 48 is provided in the middle of the pipe 54.

The injection valve 50 is a valve that can collect a certain amount of sample for introduction and introduce the sample for introduction into the analytical column 44. The injection valve 50 includes a plurality of introduction ports and discharge ports (not illustrated). In addition, an injection loop 56 is connected to the injection valve 50. The injection loop 56 can hold a certain amount of liquid. By appropriately switching the injection valve 50, it is possible to select a state in which the injection loop 56 communicates with the dilution tank 42 and the sample for introduction is supplied from the dilution tank 42 to the injection loop 56 and a state in which the injection loop 56 communicates with the analytical column 44 via a pre-filter PF and a pipe 58 and the sample for introduction is introduced from the injection loop 56 to the analytical column 44.

The photometric unit 38 is a device configured to optically detect hemoglobin contained in a desorption liquid from the analytical column 44. The photometric unit 38 is connected to a waste liquid tank 60 configured to discharge the desorption liquid from the analytical column 44 via the pipe 58.

Fig. 2 is a block diagram of a control system of the analysis apparatus 30. As illustrated in Fig. 2, the analysis apparatus 30 includes a control unit 70. The control unit 70 includes a central processing unit (CPU) 70A, a read only memory (ROM) 70B, a random access memory (RAM) 70C, a nonvolatile memory 70D, an input/output interface (I/O) 70E, and a bus 70F. The CPU 70A, the ROM 70B, the RAM 70C, the nonvolatile memory 70D, and the input/output interface 70E are connected to each other via the bus 70F.

Each of the sample preparation unit 34, the analysis unit 36, and the photometric unit 38 is connected to the input/output interface 70E.

Note that a liquid chromatography measurement program to be described later is stored in advance in the nonvolatile memory 70D as an example in the embodiment. The CPU 70A reads and executes the liquid chromatography measurement program stored in the nonvolatile memory 70D. In addition, the liquid chromatography measurement program may be recorded in a recording medium, for example, a CD-ROM or the like, and may be read by a CD-ROM drive or the like to be executed.

In addition, an external communication unit 72, a barcode reader 74, an operation unit 76, and a display unit 78 are connected to the control unit 70, respectively.

The external communication unit 72 is a device configured to communicate with the outside via a network line.

The barcode reader 74 reads a barcode attached to the blood collection tube 100 containing the blood sample 102 as the specimen. Information on the barcode read by the barcode reader 74 is transmitted to the management apparatus 80, which will be described later, through the external communication unit 72. The management apparatus 80 selects a measurement mode to be performed regarding the blood sample 102 based on the barcode information on the blood collection tube 100, and transmits the measurement mode to the control unit 70 (CPU 70A) through the external communication unit 72. The barcode information is information specific to the specimen, and is, for example, a specimen ID.

The operation unit 76 is a portion that receives an input from a test worker.

The display unit 78 displays an operation content of the operation unit 76, a measurement mode to be described later, and the like. In addition, a chromatogram drawn based on a detection result in the photometric unit 38 may be displayed.

In addition, the analysis apparatus 30 has at least two measurement modes. Specifically, the analysis apparatus 30 has a first measurement mode and a second measurement mode, and is configured to switch between the first measurement mode and the second measurement mode.

The first measurement mode is a mode of more rapidly measuring the specimen as compared with the second measurement mode. Here, examples of "more rapidly measuring" include a decrease in time (measurement time) until a measurement result is acquired as compared with the second measurement mode and the like. Examples of a factor of the decrease in measurement time of the specimen include acquisition of a measurement result as simple information (when the measurement result is numerical data, a small number of digits after the decimal point of the numerical data is small or the like), a small number of measurement items, and the like.

The second measurement mode is a mode of measuring the specimen in more detail as compared with the first measurement mode. Here, examples of "measuring in more detail" include including a measurement item different from that in the first measurement mode, an increase in measurement time of the specimen as compared with the first measurement mode, and the like. Examples of a factor of the increase in time until the measurement result is acquired include acquisition of a measurement result as detailed information (when the measurement result is numerical data, a large number of digits after the decimal point of the numerical data) and the like.

Note that, in the analysis apparatus 30 of the embodiment, a fast mode is set as an example of the first measurement mode, and a variant mode is set as an example of the second measurement mode.

Here, the variant mode is a detailed measurement mode in which HbA1c and variant hemoglobin in the blood sample 102 are measured as measurement items by sequentially feeding a plurality of types of eluents, which will be described later, to the analytical column 44 in a predetermined order using liquid chromatography. In the variant mode of the embodiment, for example, HbA1c, HbA0, HbF, HbS, and HbC can be measured.

In addition, the fast mode is a mode in which HbA1c is measured by sequentially feeding a plurality of common eluents, common to the eluents used in the variant mode among a plurality of types of eluents, to the analytical column 44 in a predetermined order, and is a rapid measurement mode specialized for mainly measuring HbA1c as a measurement item. In the fast mode of the embodiment, for example, HbA1c, HbA0, and HbF can be measured.

That is, the variant mode is the detailed measurement mode that includes a measurement item different from that in the fast mode. Specifically, the variant mode has more measurement items than the fast mode, and thus, the measurement time becomes longer.

### (Management Apparatus 80)

Fig. 2 illustrates a schematic configuration of the management apparatus 80.

The management apparatus 80 is an apparatus that can manage at least one analysis apparatus 30. Note that the management apparatus 80 is an example of a management unit of the invention.

Fig. 2 illustrates a block diagram of a control system of the management apparatus 80. As illustrated in Fig. 2, the management apparatus 80 includes a central processing unit (CPU) 80A, a read only memory (ROM) 80B, a random access memory (RAM) 80C, a nonvolatile memory 80D, and a bus 80E. The CPU 80A, the ROM 80B, the RAM 80C, and the nonvolatile memory 80D are connected via the bus 80E, respectively.

Note that a specimen measurement mode selection program to be described later is stored in advance in the nonvolatile memory 80D as an example in the embodiment. The CPU 80A reads and executes the specimen measurement mode selection program stored in the nonvolatile memory 80D. In addition, for example, the specimen measurement mode selection program may be recorded in a recording medium, for example, a CD-ROM or the like, and may be read by a CD-ROM drive or the like to be executed.

In addition, each of an external communication unit 82, an operation unit 84, a display unit 86, and a database 88 is connected to the management apparatus 80.

The external communication unit 82 is a device configured to communicate with the outside via a network line. The management apparatus 80 can communicate with the analysis apparatus 30 through the external communication unit 82. Note that the network line here may be an in-house information communication network (LAN) constructed in a medical institution in which the analysis apparatus 30 and the management apparatus 80 are installed, or may be a communication line such as the Internet.

The operation unit 84 is a portion that receives an input from an operator.

The display unit 86 displays an operation content of the operation unit 84, a measurement mode to be described later, and the like.

The database 88 stores information (hereinafter, appropriately referred to as "test information") regarding a test content of the blood sample 102. Note that the test content (referred to as a "measurement item") of the blood sample 102 here refers to a component contained in the blood sample 102 to be measured. In addition, the test information on the blood sample 102 is stored in association with barcode information (information on a barcode attached to the blood collection tube 100) corresponding to the blood sample 102.

The test information on the blood sample 102 includes information (hereinafter appropriately referred to as "clinical department information") on a clinical department that has requested a blood collection room in a hospital for a blood collection test of a patient. Note that the clinical department information may include not only information on a clinical department in the same hospital but also information on a clinical department of a different hospital such as a branch hospital.

Note that the test information on the blood sample 102 is transmitted to the management apparatus 80 as a worker belonging to the clinical department requesting the blood collection test inputs via a support system such as a laboratory information management system provided by an external apparatus 190, and is stored in the database 88.

In addition, the database 88 stores information in which the clinical department information is associated with a measurement mode. For example, the database 88 stores that measurement is performed in the variant mode in a case in which the clinical department information is a department of diabetes internal medicine, and that measurement is performed in the fast mode in a case in which the clinical department information is a medical examination department. The information in which the clinical department information is associated with the measurement mode may be arbitrarily input by a user through the operation unit 84.

When an inquiry about a measurement mode of the blood sample 102 is received from the analysis apparatus 30 (in other words, the barcode information on the blood sample 102 is received), the CPU 80A selects the measurement mode of the blood sample 102 from the fast mode and the variant mode based on the information, stored in the database 88, in which the clinical department information is associated with the measurement mode. Then, the selected measurement mode is transmitted to the analysis apparatus 30.

Next, an operation of analyzing the blood sample 102 performed by the analysis apparatus 30 will be described.

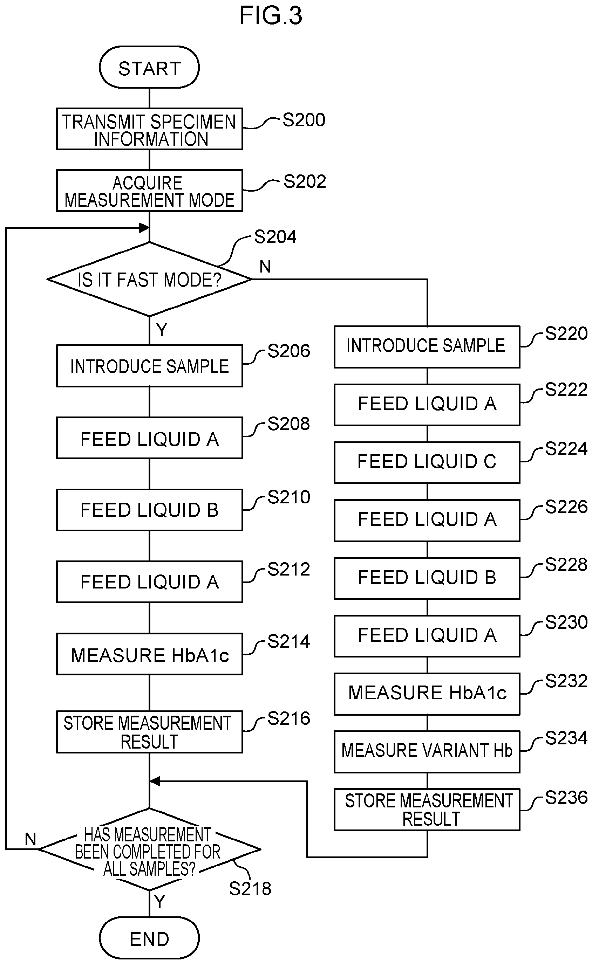

Fig. 3 illustrates an example of a flowchart of liquid chromatography measurement processing executed by the CPU 70A of the control unit 70.

First, the test worker sets the blood collection tube 100 containing the blood sample 102 in the analysis apparatus 30. Specifically, a rack 104 accommodating a plurality of (for example, ten) blood collection tubes 100 is set in the analysis apparatus 30. The set rack 104 is conveyed to a collection position where the blood sample 102 is collected from the blood collection tube 100 by a conveying apparatus (not illustrated) included in the analysis apparatus 30.

When the test worker instructs the analysis apparatus 30 to start measurement, the CPU 70A reads and executes the liquid chromatography measurement program stored in the nonvolatile memory 70D.

In step S200, barcode information read from a barcode of the blood collection tube 100 by the barcode reader 74 is transmitted to the management apparatus 80. Note that, in the embodiment, the barcodes of all the blood collection tubes 100 are continuously read by the barcode reader 74, and pieces of the read information are transmitted to the management apparatus 80. However, the barcode of the blood collection tube 100 may be read by the barcode reader 74 every time the blood sample 102 is analyzed.

In step S202, a measurement mode of the blood sample 102 is acquired from the management apparatus 80. Note that the measurement modes of all the blood samples 102 are acquired from the management apparatus 80 in the embodiment, but the measurement mode may be acquired from the management apparatus 80 every time the blood sample 102 is analyzed.

In step S204, it is determined whether or not the measurement mode acquired from the management apparatus 80 is the fast mode. In the case of the fast mode, the processing proceeds to step S206. In a case in which the measurement mode acquired from the management apparatus 80 is not the fast mode, the processing proceeds to step S220.

A plurality of types of eluents used in the embodiment are liquid A, liquid B, and liquid C. The liquid A is, for example, an eluent for eluting HbA1c and for equilibrating the analytical column 44. The liquid B is, for example, an eluent for eluting all the hemoglobin remaining in the analytical column 44, that is, an eluent for washing the analytical column 44. The liquid C is, for example, an eluent for eluting hemoglobin other than HbA1c after elution of HbA1c. Note that eluting power of hemoglobin is higher in the order of the liquid B, the liquid C, and the liquid A.

In the variant mode, measurement is performed by feeding the liquid A, the liquid B, and the liquid C to the analytical column 44 in a predetermined order. In the fast mode, measurement is performed by feeding the liquid A and the liquid B to the analytical column 44 in a predetermined order. That is, the liquid A and the liquid B are common eluents commonly used in the variant mode and the fast mode, and the liquid C is a non-common eluent used only in the variant mode.

In step S206, the blood sample 102 is introduced. Specifically, the blood sample 102 is collected from the blood collection tube 100 by operating the nozzle 40. The blood sample 102 collected by the nozzle 40 is supplied to the dilution tank 42 by operating the nozzle 40.

Then, the sample held in the injection loop 56 by switching the injection valve 50 is filtered by the pre-filter PF and introduced into the analytical column 44. When the sample is introduced into the analytical column 44, HbA1c, variant Hb, and the like are adsorbed to the packing material.

In step S208, the liquid A is supplied to the analytical column 44 for a predetermined time. As a result, HbA1c is eluted from the analytical column 44.

In step S210, the liquid B is supplied to the analytical column 44 for a predetermined time. As a result, all the hemoglobin remaining in the analytical column 44 is eluted, and the analytical column 44 is washed.

In step S212, the liquid A is supplied to the analytical column 44 for a predetermined time. As a result, the analytical column 44 is equilibrated.

As described above, the feeding to the analytical column 44 is performed in the order of "liquid A → liquid B → liquid A" in the fast mode.

A desorption liquid containing various types of hemoglobin discharged from the analytical column 44 is supplied to the photometric unit 38 via the pipe 58. This desorption liquid is guided to the waste liquid tank 60 via the pipe 58.

In the photometric unit 38, the desorption liquid is continuously irradiated with light, and a light reception result (absorbance) thereof is output to the control unit 70. Then, the control unit 70 calculates a chromatogram.

The chromatogram is a graph showing a relationship between an elapsed time from the start of measurement and absorbance as the light reception result. It is possible to know which hemoglobin has been detected based on any position at which a peak of this chromatogram appears, and it is possible to know the concentration of hemoglobin based on an integrated value of the absorbance at peak portions (mountain portions), that is, a size of areas of the peak portions.

In step S214, HbA1c is measured based on the calculated chromatogram.

In step S216, a measurement result is stored in the nonvolatile memory 70D. That is, the concentration of HbA1c and the presence or absence of detection of variant hemoglobin are stored in the nonvolatile memory 70D.

Whether or not the measurement has been completed for all the blood collection tubes 100 is determined in step S218. In a case in which the measurement has been completed for all the blood collection tubes 100, this routine is terminated. In a case in which the blood collection tube 100 for which the measurement has not been completed is present, the processing returns to step S204, and it is determined whether or not the measurement mode acquired from the management apparatus 80 for the blood sample 102 to be measured is the fast mode.

In a case in which it is determined in step S204 that the measurement mode is not the fast mode, that is, the measurement is to be performed in the variant mode, the processing proceeds to step S220.

In step S220, the blood sample 102 is introduced as in step S206.

In step S222, the liquid A is supplied to the analytical column 44 for a predetermined time. As a result, HbA1c is eluted from the analytical column 44.

In step S224, the liquid C is supplied to the analytical column 44 for a predetermined time. As a result, hemoglobin other than HbA1c is eluted after HbA1c is eluted from the analytical column 44.

In step S226, the liquid A is supplied to the analytical column 44 for a predetermined time. As a result, the analytical column 44 is equilibrated.

In step S228, the liquid B is supplied to the analytical column 44 for a predetermined time. As a result, all the hemoglobin remaining in the analytical column 44 is eluted, and the analytical column 44 is washed.

In step S230, the liquid A is supplied to the analytical column 44 for a predetermined time. As a result, the analytical column 44 is equilibrated.

As described above, the feeding to the analytical column 44 is performed in the order of "liquid A → liquid C → liquid A → liquid B → liquid A" in the variant mode.

In step S232, HbA1c is measured as in step S214.

In step S234, variant hemoglobin is measured based on a calculated chromatogram.

In step S236, a measurement result is stored in the nonvolatile memory 70D. That is, the concentration of HbA1c and the concentration of variant hemoglobin are stored in the nonvolatile memory 70D.

Accordingly, the measurement can be performed by switching between the fast mode and the variant mode using one apparatus based on an instruction from the management apparatus 80 in the embodiment.

Next, a flow of selection of a measurement mode of the blood sample 102 by the management apparatus 80 will be described.

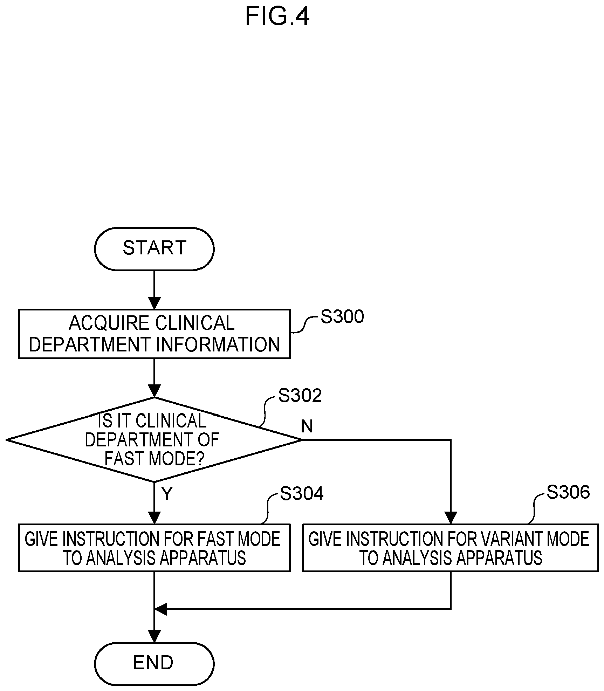

Fig. 4 illustrates an example of a flowchart of specimen measurement mode selection processing executed by the CPU 80A of the management apparatus 80.

When information (barcode information) regarding the blood sample 102 is received from the analysis apparatus 30, the CPU 80A reads and executes the specimen measurement mode selection program stored in the nonvolatile memory 80D.

In step S300, the CPU 80A accesses the database 88 based on the barcode information on the blood sample 102, and specifies information (clinical department information) on a clinical department that has requested a blood collection test corresponding to the barcode information.

In step S302, the CPU 80A determines whether a measurement mode of the blood sample 102 is the fast mode or the variant mode based on information, stored in the database 88, in which the clinical department information is associated with the measurement mode. In a case in which it is determined that the measurement mode of the blood sample 102 is the fast mode, the processing proceeds to step S304. In a case in which it is determined that the measurement mode of the blood sample 102 is not the fast mode, the processing proceeds to step S306.

In step S304, the CPU 80A transmits an instruction to perform measurement in the fast mode to the analysis apparatus 30 through the external communication unit 82.

In step S306, the CPU 80A transmits an instruction to perform measurement in the variant mode to the analysis apparatus 30 through the external communication unit 82.

Accordingly, the instruction to perform measurement in the selected measurement mode is transmitted from the management apparatus 80 to the analysis apparatus 30 in the embodiment.

Next, an operational effect of the embodiment will be described.

In a medical institution, a specimen test such as analysis of hemoglobin in blood is sometimes performed in a clinical examination department which is specialized in specimen analysis and in which various analysis apparatuses are installed. In such a medical institution, a test content (measurement item) is sometimes defined in each clinical department (a department of internal medicine, a department of diabetes internal medicine, a medical examination department, or the like) that is a test request source. That is, in the analysis of hemoglobin in blood, whether measurement is performed in the fast mode or in the variant mode is sometimes defined by a clinical department (type of patient who visits a hospital). For example, in the department of diabetes internal medicine, a measured value of HbA1c is important in diagnosis and treatment of diabetes. However, in a case in which the patient has variant hemoglobin, there is a possibility that a measurement result of HbA1c is affected. Thus, in many cases, HbA1c and variant hemoglobin are separated, and measurement is performed in the variant mode in which a measured value of HbA1c is hardly affected. Meanwhile, since the measured value of HbA1c is required as a screening test in the medical examination department, measurement is performed in the fast mode, and a retest is performed in a specialized department when detailed measurement in the variant mode is required from a measurement result in the fast mode, and thus, the measurement is performed only in the fast mode in many cases. Further, specimens having different test contents are delivered in a mixed manner to a laboratory where the specimens are tested. A worker in the laboratory sets a measurement mode of an analysis apparatus according to a test content of a specimen. In a case in which the measurement mode is set according to the test content of the specimen as described above, it is necessary to pay attention to prevent occurrence of a setting error, and a burden on the worker tends to increase.

On the other hand, in the analysis system 20 of the embodiment, even in a case in which specimens having different test contents exist in a mixed manner, that is, even in a case in which the blood collection tubes 100 measured in the fast mode and the variant mode are accommodated in a mixed manner in the rack 104, the management apparatus 80 automatically selects a measurement mode of the analysis apparatus 30 based on information in which the clinical department information on a clinical department that has requested a test set in the blood sample 102 is associated with the measurement mode. Therefore, in the analysis system 20, for example, the burden on the test worker is mitigated as compared with a case in which the test worker sets the measurement mode each time according to the test content required by the clinical department that has requested the test.

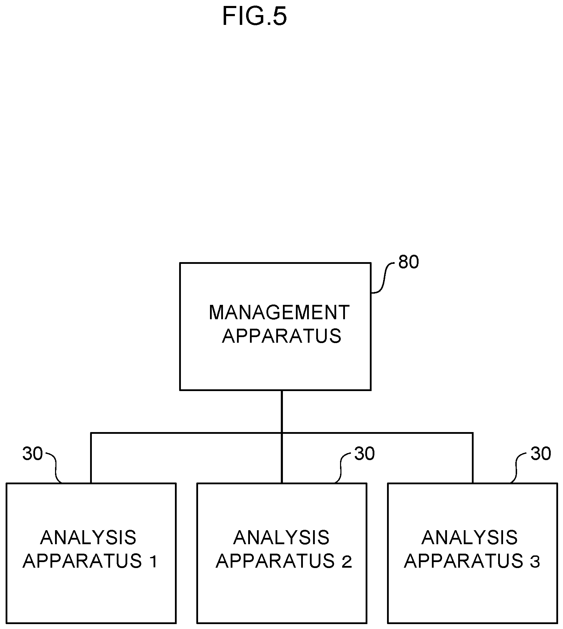

Although the management apparatus 80 and one analysis apparatus 30 are connected via the network line in the embodiment, the invention is not limited to such a configuration. For example, as illustrated in Fig. 5, a plurality of the analysis apparatuses 30 may be connected to the management apparatus 80 via the network line. Note that three analysis apparatuses 30 are connected to the management apparatus 80 through the network line in the example of Fig. 5.

Although the management apparatus 80 and the analysis apparatus 30 are connected via the network line in the embodiment, the invention is not limited to such a configuration. The control unit 70 of the analysis apparatus 30 may have the function of the management apparatus 80. In this case, it is preferable to provide the database 88 in the analysis apparatus 30.

In addition, the clinical department information is used as the information regarding the test content of the blood sample 102 in the embodiment, but the invention is not limited to such a configuration. For example, the clinical department information may be associated with information on a doctor belonging to a clinical department. In this case, information on a clinical department to which a doctor belongs can be acquired from information on the doctor who has requested a blood collection test of a patient. Note that the information on the doctor is identification information of the doctor, and is, for example, a barcode of a unique ID of the doctor or the like. As an example, measurement is performed in a fast mode in the case of a test request from a doctor belonging to the medical examination department, and measurement is performed in the variant mode in the case of a test request from a doctor belonging to the department of diabetes internal medicine.

Although the feeding to the analytical column 44 is performed in the order of "liquid A → liquid B → liquid A" in the fast mode of the embodiment and in the variant mode and the feeding to the analytical column 44 is performed in the order of "liquid A → liquid C → liquid A → liquid B → liquid A", the invention is not limited to such a configuration. For example, a solvent having higher eluting power may be used in the fast mode as compared with the variant mode. As a result, the measurement time in the fast mode can be shortened.

A feeding speed of a solvent may be increased in the fast mode as compared with the variant mode. Specifically, in a case in which the fast mode is executed, the feeding speed may be increased by increasing a rotation speed of the liquid feeding pump 48 as compared with a case in which the variant mode is executed. As a result, the measurement time in the fast mode can be shortened.

An eluent may be switched to an eluent having higher eluting power and measurement may be continued in a case in which a target component is detected in the fast mode, and measurement may be continued without switching an eluent even in a case in which a target component is detected in the variant mode. As a result, the measurement time in the fast mode can be shortened.

Although the analysis apparatus 30 using high performance liquid chromatography is managed by the management apparatus 80 in the embodiment, the invention is not limited to such a configuration. For example, a blood cell measurement apparatus that measures cell types in blood may be managed by the management apparatus 80. That is, the analysis system of the invention may be applied to an apparatus that measures cell types in blood. Note that only a normal cell type among cell types in blood is measured as a measurement item in the first measurement mode of the blood cell measurement apparatus, and an abnormal cell different from the normal cell type among the cell types in blood is further measured as a measurement item in the second measurement mode.

Furthermore, the invention is not limited to the above description. Needless to say, various modifications can be made that fall within a scope of the invention, as defined by the claims.

The following clauses set out features of the invention which may not presently be claimed in this application, but which may form the basis for future amendment or a divisional application.
1. An analysis system including:
   an analyzer that analyzes a specimen, the analyzer being switchable between a first measurement mode and a second measurement mode that includes a measurement item different from a measurement item of the first measurement mode; and
   a management unit that selects a measurement mode from the first measurement mode and the second measurement mode of the analyzer based on information on a clinical department that has requested a test to be set for the specimen.
2. The analysis system according to clause 1, in which the information on the clinical department is associated with information on a doctor belonging to the clinical department.
3. The analysis system according to clause 1 or 2, wherein the analyzer is configured to analyze the specimen by liquid chromatography.
4. The analysis system according to clause 3, in which
   hemoglobin A1c in the specimen is measured in the first measurement mode, and
   variant hemoglobin in the specimen is measured in the second measurement mode.
5. The analysis system according to clause 3 or 4, in which a solvent having higher eluting power is used in the first measurement mode as compared with the second measurement mode.
6. The analysis system according to any one of clauses 3 to 5, in which a feeding speed of a solvent is increased in the first measurement mode as compared with the second measurement mode.
7. The analysis system according to any one of clauses 3 to 6, in which
   in the first measurement mode, in a case in which a target component is detected, a solvent is switched to a solvent having higher eluting power and measurement is continued, and
   in the second measurement mode, measurement is continued without switching a solvent even in a case in which a target component is detected.

## Claims

1. An analysis system (20), comprising:
an analyzer (30) configured to analyze a specimen (102), the analyzer (30) being switchable between a first measurement mode and a second measurement mode that includes a measurement item different from a measurement item of the first measurement mode; and
a management unit (80) configured to select a measurement mode from the first measurement mode and the second measurement mode of the analyzer (30) based on information on a clinical department that has requested a test to be set for the specimen (102).

2. The analysis system (20) according to claim 1, wherein the information on the clinical department is associated with information on a doctor belonging to the clinical department.

3. The analysis system (20) according to claim 1 or 2, wherein the analyzer (30) is configured to analyze the specimen (102) by liquid chromatography.

4. The analysis system (20) according to any preceding claim, wherein
hemoglobin A1c in the specimen (102) is measured in the first measurement mode, and
variant hemoglobin in the specimen (102) is measured in the second measurement mode.

5. The analysis system (20) according to claim 3, or claim 4 when dependent from claim 3, wherein a solvent having higher eluting power is used in the first measurement mode as compared with the second measurement mode.

6. The analysis system (20) according to claim 3 or 5, or claim 4 when dependent from claim 3, wherein a feeding speed of a solvent is increased in the first measurement mode as compared with the second measurement mode.

7. The analysis system (20) according to claim 3, 5 or 6, or claim 4 when dependent from claim 3, wherein
in the first measurement mode, in a case in which a target component is detected, a solvent is switched to a solvent having higher eluting power and measurement is continued, and
in the second measurement mode, measurement is continued without switching a solvent even in a case in which a target component is detected.
